## Europäisches Patentamt
## European Patent Office
## Office européen des brevets

(19)

(11) Veröffentlichungsnummer: **0 218 005**
**B1**

(12) # EUROPÄISCHE PATENTSCHRIFT

(45) Veröffentlichungstag der Patentschrift:
23.11.88

(51) Int. Cl.⁴: **C 07 C 143/675,** C 07 C 143/48,
C 07 C 139/14

(21) Anmeldenummer: **86107038.1**

(22) Anmeldetag: **23.05.86**

(54) Eintopfverfahren zur Herstellung von 2-Acetaminonaphthalin-6-sulfonsäure hoher Reinheit.

(30) Priorität: **09.10.85 DE 3536036**

(43) Veröffentlichungstag der Anmeldung:
**15.04.87 Patentblatt 87/16**

(45) Bekanntmachung des Hinweises auf die Patenterteilung:
**23.11.88 Patentblatt 88/47**

(84) Benannte Vertragsstaaten:
**CH DE FR GB IT LI**

(56) Entgegenhaltungen:
**EP-A-0 031 299**
**DE-A-2 831 965**
**DE-A-3 219 651**
**DE-B-2 531 281**
**GB-A-436 464**
**GB-A-1 341 351**
**GB-A-2 001 645**
**GB-A-2 137 621**
**US-A-4 321 215**

**SOVIET INVENTIONS ILLUSTRATED, Sektion Ch, Woche E 17; 9.Juni 1982 DERWENT PUBLICATIONS LTD., London, E 12**
**SOVIET INVENTIONS ILLUSTRATED, Sektion Ch, Woche C 06, 19.März 1980 DERWENT PUBLICATIONS LTD., London, E14**

(73) Patentinhaber: **HOECHST AKTIENGESELLSCHAFT, Postfach 80 03 20, D-6230 Frankfurt am Main 80 (DE)**

(72) Erfinder: **Arndt, Otto., Dr., Frankfurter Strasse 38, D-6238 Hofheim (DE)**
Erfinder: **Papenfuhs, Theodor, Dr., Heinrich- Bleicher- Strasse 40, D-6000 Frankfurt/M. 50 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein Eintopfverfahren zur Herstellung von 2-Acetaminonaphthalin-6-sulfonsäure bzw. der nichtacetylierten Vorstufe (2-Aminonaphthalin-6-sulfonsäure) hoher Reinheit.

Die Herstellung von 2-Aminonaphthalin-6-sulfonsäure mit Ammoniak in Gegenwart von Ammoniumbisulfit aus 2-Hydroxynaphthalin-6-sulfonsäure (Bucherer-Reaktion) ist beispielsweise aus den folgenden Literaturstellen bekannt: ORGANIC REACTIONS 1 (1942), N. Drake, Seite 105; AROMATIC REARRANGEMENTS, H. Shine, Elsevier Publ. Company, 1967, Seite 207; ANGEWANDTE CHEMIE 79 (1967), Nr. 8, Seiten 329 - 388.

Nach diesen Verfahren wird ausnahmslos eine 2-Aminonaphthalin-6-sulfonsäure erhalten, die durch unerwünschte Nebenkomponenten, insbesondere durch 2-Aminonaphthalin, verunreinigt ist.

Die Aufgabenstellung bestand somit darin, geeignete Maßnahmen in prinzipiell bekannten Verfahren zur Herstellung von 2-Acetaminonaphthalin-6-sulfonsäure bzw. von deren nichtacetylierter Vorstufe (2-Aminonaphthalin-6-sulfonsäure) zu finden, um den Gehalt an Nebenkomponenten, insbesondere 2-Aminonaphthalin, zu minimieren.

In der DE-AS-2 531 281 wird ein kontinuierliches Herstellungsverfahren auch für die 2-Aminonaphthalin-6-sulfonsäure beschrieben, bei welchem auf das Auftreten von Aminonaphthalin - Derivaten in den Abgasen der diskontinuierlich betriebenen Verfahrensweise hingewiesen wird. Für das Verfahren wurden die reinen Alkalimetall- oder Ammoniumsalze der 2-Hydroxynaphthalin-6-sulfonsäure eingesetzt, d.h. es wird nicht vom 2-Hydroxynapthalin ausgegangen. Im übrigen handelt es sich um kein Eintopfverfahren.

In der Britischen Patentschrift 436 464 wird ein Eintopfverfahren zur Herstellung von 2-Aminonaphthalin-6-sulfonsäure ("Brönner-Säure"), jedoch ohne Berücksichtigung des Auftretens des 2-Aminonaphthalins beachrieben.

Schließlich wird in der EP-PS-0 031 299 ein Verfahren zur Herstellung von 2-Aminonaphthalin-1-sulfonsäure ("Tobias-Säure"), ausgehend vom 2-Hydroxynaphthalin, beschrieben mit der Zielsetzung, diese Säure mit einem nur sehr geringen Gehalt an 2-Aminonaphthalin in hoher Raum-Zeit-Ausbeute herstellen zu können. Das Ziel, den Gehalt an 2-Aminonaphthalin in der Tobias-Säure auf Spuren (unter 0,1 Gewichtsprozent) herabzusetzen, wird hierbei durch Extraktion mit organischen Lösemitteln an zwei Stellen des Verfahrens und durch Umstellung von Alkalimetallsalzen auf die leichter löslichen Ammoniumsalze in den Zwischenstufen erreicht. Ein wesentlicher Nachteil dieses Verfahrens besteht darin, daß die Extraktionen in zwei Stufen erfolgen. Zählt man den bei der Sulfonierung des 2-Hydroxynaphthalins mit Chlorsulfonsäure als Verdünnungsmittel verwendeten chlorierten Kohlenwasserstoff mit, dessen Phase nach der Neutralisation der gebildeten Sulfonsäure von der wäßrigen Produktphase abgetrennt wird, so handelt es sich sogar um drei Extraktionsstufen.

Die erste Extraktion ist hierbei dadurch gekennzeichnet, daß sie auf der Stufe des Ammoniumsalzes der 2-Hydroxynaphthalin-1-sulfonsäure (Oxy-Tobias-Säure-Ammoniumsalzlösung), also nach der Sulfonierung und Neutralisation erfolgt, und daß hierbei 2-Hydroxynaphthalin mit chlorierten Kohlenwasserstoffen, besonders 1,2-Dichlorethan, bei 70° C bis 75° C in einer Gegenstromextraktionskolonne extrahiert wird. Die zweite Extraktion ist dadurch gekennzeichnet, daß sie auf der Stufe des Natriumsalzes der 2-Aminonaphthalin-1-sulfonsäure (Tobiassäure Natrium-Salzlösung), also nach der Bucherer-Reaktion erfolgt, und daß hierbei 2-Aminonaphthalin mit Toluol oder Xylol bei 70 - 75° C in einer Gegenstromextraktionskolonne extrahiert wird.

Die Anwendung von chlorierten Kohlenwasserstoffen ist ökologisch sehr bedenklich und erfordert einen großen technischen Aufwand und exakte Produktmengenkontrollen, um eine Belastung der Umwelt (Abwasser, Abluft) zu vermeiden. Offenbar reicht hier eine einzige Extraktion nicht aus, um das Ziel eines möglichst niedrigen Gehaltes an 2-Aminonaphthalin in der Tobias-Säure zu erreichen.

Die an zwei verschiedenen Stellen des Verfahrens eingreifende Reinigungsmethode durch Extraktion mit verschiedenen Lösemitteln (chlorierter Kohlenwasserstoff und Toluol/ Xylol) ist zu aufwendig. Beispielsweise müssen die einzelnen Verfahrensmaßnahmen doppelt ausgeführt werden, wie beispielsweise Anwendungen von 2 Gegenstromextraktionskolonnen, 2 Phasentrennungen und 2 Lösemittelregenerationen. Hinzu kommt, daß vor der Bucherer-Reaktion Reste von chlorierten Kohlenwasserstoffen durch Strip-Destillation im Vakuum aus der wäßrigen Oxy-Tobias-Säure-Ammoniumsalzlösung entfernt werden müssen. Erwähnt sei in diesem Zusammenhang die Möglichkeit von Korrosion im VA-Autoklav bei Ausführung der Bucherer-Reaktion in Gegenwart von abgespaltenem Chlorid.

Über die Entsorgung des aus den Toluol/Xylol-Extraktlösungen schließlich bei der Lösemittelregeneration anfallenden 100 %-igen 2-Aminonaphthalins wird keine Aussage gemacht. Eine Verbrennung des Lösemittels wäre zu teuer. Die in der zitierten EP-PS für die erste Extraktion angegebene Regenerationsmethode für den chlorierten Kohlenwasserstoff durch Extraktion mit Wasser dürfte angesichts des Vorliegens von Nebenprodukten wie Kresole, Naphthalin, Dimethylphenol, Methylnaphthalin, Methylnaphthole, Phenylnaphthalin, 2,2'-Dihydroxy-1,1'-dinaphthyl, polycyclische Kohlenwasserstoffe (wie beispielsweise $C_{18}H_{12}$) oder Dinaphthylether nicht ausreichen. Eine destillative Regeneration dürfte daher unumgänglich sein.

Ein weiterer Nachteil dieses Verfahrens ist, daß die Sulfierung (mit Chlorsulfonsäure) in einem organischen Lösemittel (chlorierter Kohlenwasserstoff) erfolgt und dieses Lösemittel einen dritten Lösemittel-Recycling-Kreislauf nötig macht (Extraktion mit Wasser, entwässern). Das Verfahren setzt eine erhebliche Löslichkeit der Salze der Hydroxy- bzw. Aminonaphthalinsulfonsäuren in Wasser voraus. Es ist auf andere isomere Hydroxy- bzw. Aminonaphthalinsulfonsäuren, deren Salze deutlich schwerer löslich sind, nicht übertragbar, wenn sie nur in Form von Suspensionen vorliegen und somit die Phasentrennung nach Extraktion mit organischem Lösemittel unmöglich machen.

Die durch die zitierten Literaturstellen gegebenen Möglichkeiten waren somit für eine Lösung der gestellten Aufgabe, Herstellung von 2-Aminonaphthalin-6-sulfonsäure bzw. deren N-Acetylderivat mit einem möglichst niedrigen Gehalt an 2-Aminonaphthalin, nicht brauchbar.

Die gestellte Aufgabe wurde prinzipiell dadurch gelöst, daß bei der Sulfonierung von 2-Hydroxynaphthalin nicht umgesetzte (d.h. nicht sulfonierte) Verbindung (2-Hydroxynaphthalin) an geeigneten Stellen des Herstellungsverfahrens vor der Bucherer-Reaktion bis auf einen Spurenbereich von < 20 ppm entfernt wird, weil das 2-Aminonaphthalin aus vorhandenem 2-Hydroxynaphthalin durch Bucherer-Reaktion entsteht.

Gegenstand der Erfindung ist ein Eintopfverfahren zur Herstellung von 2-Acetaminonaphthalin-6-sulfonsäure hoher Reinheit d.h. unter weitestgehender Vermeidung von Verunreinigungen, insbesondere von 2-Aminonaphthalin, im Endprodukt 2-Acetylaminonaphthalin-6-sulfonsäure durch Sulfonierung von 2-Hydroxynaphthalin mit konzentrierter Schwefelsäure, Überführung der gebildeten 2-Hydroxynaphthalinsulfonsäure mit Ammoniak in Gegenwart von Ammoniumhydrogensulfit in die 2-Aminonaphthalin-6-sulfonsäure (Bucherer-Reaktion) und deren Acetylierung zur 2-Acetaminonaphthalin-6-sulfonsäure, dadurch gekennzeichnet, daß man nach Verdünnen der Sulfierschmelze mit Wasser in der angefallenen wässrigen Lösung der 2-Hydroxynaphthalin-6-sulfonsäure noch enthaltenes, nicht umgesetztes 2-Hydroxynaphthalin durch Extraktion mit Toluol oder Xylol und/oder Klären über Aktivkohle weitestgehend (< 20 ppm) entfernt.

Das erfindungsgemäße Verfahren ermöglicht die Herstellung von 2-Acetaminonaphthalin-6-sulfonsäure und 2-Aminonaphthalin-6-sulfonsäure, wobei kein 2-Aminonaphthalin extrahiert werden muß, wodurch diese Verfahrensstufe entfällt. Die isolierte 2-Aminonaphthalin-6-sulfonsäure (Brönner-Säure) enthält weniger als 100 ppm 2-Aminonaphthalin. Ihre wäßrige Suspensionen enthalten weniger als 20 ppm 2-Aminonaphthalin.

Entgegen den aus der zitierten EP-PS-0 031 299 ableitbaren Vorauasetzungen erwies sich überraschenderweise eine einzige Verfahrensstufe als ausreichend, sofern man das restliche 2-Hydroxynaphthalin nach der Sulfonierung aus dem mit Wasser verdünnten, nicht neutralisierten Sulfiergemisch extrahiert und/oder an Aktivkohle adsorbiert. Für die Extraktion sind aromatische Lösemittel, wie Toluol und Xylol, also technisch gut handhabbare Lösemittel, geeignet.

Es muß als erstaunlich und überraschend angesehen werden, daß für denselben Effekt, wie er in der zitierten EP-PS beschrieben wird, nur 1 Extraktion statt 2 Extraktionen erforderlich ist, offenbar dadurch bedingt, daß sie an einer geeigneteren Stelle des Verfahrensablaufs eingreift. Die Sulfierung des 2-Hydroxynaphthalins wird ohne Verdünnungsmittel mit Schwefelsäure gemäß beispielsweise BIOS Final Report 986, Seite 388, GB-PS-1 341 351 oder J.Soc.Chem.Ind. 46, (1927), 25 T - 27 T ausgeführt. Das Abdampfen von Wasser am Ende der Sulfierung verringert die Restmenge 2-Naphthol.

Nachstehend seien noch Einzelheiten bzw. bevorzugte Ausführungen des erfindungsgemäßen Verfahrens beschrieben:

2-Hydroxynaphthalin wird mit der gleichen Gewichtsmenge 96 %-iger Schwefelsäure (1,41 Mol $H_2SO_4$ pro Mol 2-Hydroxynaphthalin) bei 86° C bis zur völligen Lösung verrührt. Dann wird stufenweise hochgeheizt und zwar zunächst auf 95° C, anschließend auf 105° C, wobei die erreichten Temperaturstufen jeweils 30 Minuten gehalten werden.

Das Reaktionsgefäß muß zu mindestens 75 % gefüllt sein und die von der Reaktionsschmelze nicht berührte obere Innenwand ebenfalls so beheizt werden, daß sich kein Sublimat von 2-Hydroxynaphthalin darauf niederschlägt. Das Abdampfen von Wasser am Ende der Sulfierung verringert die Restmenge 2-Naphthol (Beispiel 11).

Die klare Sulfierschmelze (1 Gewichtsteil) wird rasch auf 2,5 Gewichtsteile kaltes Wasser unter Rühren gegossen. Nach völligem Vermischen wird kurz zum Rückfluß geheizt. Ein Abdampfen nennenswerter Mengen Wasser ist zu vermeiden, da mit der Aufkonzentrierung eine Abspaltung von 2-Hydroxynaphthalin aus der 2-Hydroxynaphthalin-6-sulfonsäure und die Bildung von Dinaphthylether erfolgen.

Eventuell gebildete geringe Mengen Harze können gegebenenfalls durch Klären über Filterhilfsmittel (beispielweise Perlite) entfernt werden. 2-Hydroxynaphthalin wird hierbei nicht entfernt. Die Lösung enthält 0,3 Gewichtsprozent 2-Hydroxynaphthalin (entsprechend 2 % der Theorie, bezogen auf eingesetztes Hydroxynaphthalin), bzw. 0,13 Gewichtsprozent 2-Hydroxynaphthalin, falls am Ende der Sulfierung Wasser abdestilliert wurde. Das 2-Hydroxynaphthalin kann durch Klären über Aktivkohle (60 g Kohle pro Mol 2-Hydroxynaphthalinsulfonsäure) zu über 90 % entfernt werden (Verminderung des Gehalts von ca. 4700 ppm auf ca. 400 ppm). Die andere Verfahrensweise besteht in der Extraktion mit Toluol oder Xylol. Nach zehnmaligem Ausschütteln mit je ca. 20 Volumprozent Xylol bei 25°C ist in der wäßrigen Produktphase kein 2-Hydroxynaphthalin mehr nachweisbar (< 5 ppm). Untersuchungen haben gezeigt, daß für diesen Fall eine Gegenstromextraktionskolonne mit ca. 5 Stufen ausreicht (Volumenteile Speiselösung: Xylol = 2 : 1, Extraktionsausbeute 99,8 %). Besonders vorteilhaft erweist sich die Kombination von Extraktion und Behandlung mit A-Kohle, wodurch eine Gegenstromextraktionskolonne entfällt (Beispiel 11).

Die wäßrige Lösung von 2-Hydroxynaphthalin-6-sulfonsäure, die auch ca. 4 % der Theorie 2-Hydroxynaphthalin-8-sulfonsäure enthält, wird mit 25 %-igem wäßrigem Ammoniak unter Außenkühlung auf $p_H$ 7,5 gestellt (ca. 2 Mol $NH_3$ pro Mol 2-Hydroxynaphthalinsulfonsäure). Die nunmehr vorliegende Suspension wird nacheinander mit 40 %-iger Natriumhydrogensulfitlösung (ca. 1,6 Mol pro Mol 2-Hydroxynaphthalinsulfonsäure), 33 %-iger Natronlauge (ca. 1 Mol pro Mol 2-Hydroxynaphthalinsulfonsäure) und 25 %-igem wäßrigem Ammoniak (ca. 7 Mol pro Mol 2-Hydroxynapthhalin-sulfonsäure) versetzt. Die Suspension ($p_H$ 12,5) wird in einem VA-Rührautoklav auf 160°C erhitzt und 8 Stunden bei dieser Temperatur nachgerührt. Der Druck beträgt ca. 10 bar. Dann wird über eine Druckschleuse bei 20°C bis 100°C unter Stickstoffdruck 33 %-ige Natronlauge aufgedrückt (ca. 3,5 Mol/1 Mol) und 1 Stunde bei 100°C nachgerührt ($p_H$ 13,3).

Der Ansatz wird bei 20°C entspannt und das Ammoniak bei Normaldruck (102°C) möglichst vollständig abdestilliert. Das Destillat enthält kein 2-Aminonaphthalin (<5 ppm).

Die dünne Suspenaion ($p_H$ 13,4) läuft in ca. 15 Minuten unter Außenkühlung in vorgelegte ca. 30 %-ige Salzsäure (ca. 5 - 6 Mol HCl pro Mol 2-Aminonaphtalinsulfonsäure). Die dünne, hellbraune Suspension wird zum Sieden erhitzt und das Schwefeldioxid quantitativ ausgetrieben.

Die umgekehrte Arbeitsweise, Zulauf der Salzsäure zur Produktlösung, ist nicht empfehlenswert, weil in diesem Fall die Bucherer-Reaktion in Richtung Hydrolyse der Aminonaphthalinsulfonsäure zur Hydroxynaphthalinsulfonsäure ablaufen kann, namentlich dann, wenn das Ammoniak nicht weit genug abdestilliert worden war.

Man erhält eine ca. 6-7 gewichtsprozentige Suspension der 2-Aminonaphthalin-6-sulfonsäure (Brönner-Säure) mit einem Gehalt von < 5 ppm 2-Aminonaphthalin [High performance thin layer chromatography (HPTLC) mit quantitativer Auawertung durch Scanner]. Die durch Filtration abgetrennte reine Brönner-Säure fällt in einer Ausbeute von 86 % der Theorie, bezogen auf den Diazotierwert der Suspension, bzw. 70 % der Theorie, bezogen auf 2-Hydroxynaphthalinsulfonsäure, bzw. 69 % der Theorie, bezogen auf 2-Hydroxynaphthalin an. Der Gehalt an 2-Aminonaphthalin ist < 100 ppm (HPTLC, Scanner).

Der Diazotierwert des Abwassers beträgt 10 % der Theorie, bezogen auf den Diazotierwert der Suspension vor der Filtration (1,5 % Brönner-säure, 3,3 % 2-Aminonaphthalin-8-sulfonsäure und 3,1 % 2-Aminonaphthalindisulfonsäuren; HPTLC, Scanner).

Die weiter oben angegebene alternative Reinigungsmethode durch Klären mittels Aktivkohle führt zu einer Suspension der Brönner-Säure, die ca. 60 ppm 2-Aminonaphthalin enthält. Die daraus durch Filtration isolierte Brönner-Säure enthält ca. 1000 ppm 2-Aminonaphthalin.

Demnach ist die Isolierung dieser Brönner-Säure durch Filtration nicht bevorzugt. Hier bleibt jedoch die Möglichkeit, die Suspension der Acetylierung zuzuführen, d.h., das in der Suspension in geringer Menge vorhandene 2-Aminonaphthalin in das 2-N-Acetylaminonaphthalin überzuführen. Die Brönner-Säure ist ein Vorprodukt von 2-Aminonaphtha-lin-6-oxethylsulfon (CAS Nr. 52218-35-6, JA-OS 73/43 501, EP-OS-0 111 288, Beispiel 1). Die Einführung der Oxethylgruppe erfolgt über die Herstellung des Sulfochlorids der Acet-Brönner-Säure, dessen anschließende Reduktion zur Sulfinsäure mit Nariumsulfit und deren Oxethylierung mit Ethylenoxid. Für die Chlorierung zum Sulfochlorid und die Oxethylierung muß die Aminogruppe durch Acetylierung geschützt werden.

Die Acetylierung wird mit Essigsäureanhydrid (1,25 Mol pro Mol Brönner-Säure) bei 70°C und $p_H$ 7 ausgeführt. Der $p_H$ sinkt dabei auf ca. 3 ab. Man erhält isomerenfreie 2-Acetaminonaphthalin-6-sulfonsäure (Acetyl-Brönner-Säure) mit einem Gehalt von ca. 20 ppm 2-Acetaminonaphthalin (HPLC).

Wie bereits erwähnt, ist die Brönner-Säure ein Vorprodukt für 2-Aminonaphthalin-6-oxethylsulfon, das ein wertvolles Vorprodukt für solche Reaktivfarbstoffe darstellt, die sich mit einer Vinylsulfongruppe auf der Cellulosefaser verankern.

Weiterhin ist die Brönner-Säure ein Vorprodukt für Diazo- oder Kupplungskomponenten bei der Herstellung von Azofarbstoffen.

**Beispiel 1**

a) In einem Rührkolben werden 215 Teile Schwefelsäure 96 % (2,1 Mol) vorgelegt. Unter langsamem Rühren werden 216 Teile 2-Hydroxynaphthalin (1,5 Mol) in Form von Schuppen bei 30°C in ca. 30 Minuten eingetragen.

Man heizt auf genau 86°C (der Kolben soll möglichst tief im Heizbad eingetaucht sein) und rührt 30 Minuten bei dieser Temperatur bis zum völligen Verachwinden des sich langsam lösenden Bodenkörpers aus 2-Hydroxynaphthalin. Dann heizt man auf 95°C in ca. 1,5 Stunden und rührt 30 Minuten bei 95°C nach. Darauf heizt man weiter innerhalb von ca. 30 Minuten auf 105°C. Es bildet sich ein Hauch von Sublimat aus 2-Hydroxynaphthalin am obersten, kälteren Teil der Kolbeninnenwand.

Die dünnflüssige bis schwach viskose Schmelze (105°C) wird sofort anschließend in 840 Teile Wasser eingerührt, wobei sich die Temperatur auf 45°C einstellt. Der im Sulfierkolben verbliebene Anteil wird mit 150 Teilen Wasser auf einmal aufgenommen und nach kräftigem Durchrühren ebenfalls zugemischt. Man spült den Sulfierkolben mit 60 Teilen Wasser und gibt diese zum Ansatz.

Man heizt die trübe, schwarze Lösung zum Rückfluß und hält maximal 15 Minuten am Rückfluß. Im allgemeinen ist eine Klärung nicht nötig. Falls sie doch erwünscht ist, setzt man 6 Teile eines Filterhilfsmittel auf Basis eines vulkanischen Minerals (Perlite J 4) zu, kühlt auf 60°C ab, und saugt auf der Nutsche ab. (Es verbleiben 9 Teile Filterrückstand, von denen 3 Teile schwarze Harzbrocken sind).

Das ca. 22 %-ige Produktfiltrat ($d^{20}$ = 1,112, Menge ca. 1500 Teile) hat einen Gehalt von 0,30 % 2-Hydroxynaphthalin entsprechend 4,5 Teile 100 %, entsprechend 2 % der Theorie.

Die auf 25°C abgekühlte Produktlösung (1350 Volumenteile) wird 10 mal mit je 270 Volumenteilen m-Xylol ausgerührt.

Die extrahierte Produktlösung (1450 Teile) enthält 320 Teile 2-Hydroxynaphthalin-6(8)-sulfonsäure (1,42 Mol). Mit HPTLC findet man < 5 ppm 2-Hydroxynaphthalin.

Das m-Xylol (ca. 2340 Teile) wird durch Destillation über eine Kolonne (60 cm Raschigringe, Rücklauf 5 : 1, Normaldruck, Kopftemperatur 135 - 139°C) regeneriert. Der Sumpf (5 % der Ausgangsmenge = 120 Teile) enthält: 2-Hydroxynaphthalin (4,5 Teile), polycyclische Kohlenwasserstoffe, Dinaphthylether und 2,2'-Dioxy-1,1'-dinaphthyl.

6) 1450 Teile der wie vorstehend [Absatz a)] beschriebenen hergestellten wäßrigen Lösung der 2-Hydroxynaphthalin-6(8)-sulfonsäure (320 Teile = 1,42 Mol) werden mit 190 Teilen 25 %-igem wäßrigem Ammoniak (2,80 Mol) unter Außenkühlung auf $p_H$ 7,5 gestellt.

Die Suspension wird bei 5°C nacheinander mit 600 Teilen ca. 40 %-iger Natriumhydrogensulfitlösung (2,25 Mol), 182 Teilen 33 %-iger Natronlauge (1,50 Mol) und schließlich 750 Teilen 25 %-igem wäßrigem Ammoniak (11,0 Mol) versetzt.

Die Suspension ($p_H$ 12,5) wird in einem VA-Rührautoklav in ca. 40 Minuten auf 160°C erhitzt und 8 Stunden bei dieser Temperatur nachgerührt. Der Druck beträgt konstant 10 bar.

Dann werden über eine Druckschleuse bei 20°C unter Stickstoffdruck 630 Teile 33 %-ige Natronlauge (5,20 Mol) aufgedrückt. Der Ansatz wird 1 Stunde bei 100°C nachgerührt. Man kühlt auf 20°C ab, entspannt vorsichtig und entleert den Autoklav. Anschließend spült man mit 200 Teilen Wasser nach. Erhalten werden ca. 4000 Teile einer dünnen, hellbraunen Suspension der 2-Aminonaphthalin-6(8)-sulfonsäure (Salz) ($p_H$ 13,3). Darauf destilliert man ca. 870 Teile ca. 16 %-iges wäßriges Ammoniak (ca. 60 % der Theorie) bei Normaldruck bis zu einer Kopftemperatur von 100°C ab (Badtemperatur bis 150°C, Kältefalle als Vorlage). Das Ammoniak-Destillat ist durch Xylol-Tröpfchen schwach trüb und enthält gemäß HPTLC kein 2-Aminonaphthalin (< 5 ppm). Nach Aufstärken mit 105 Teilen gasförmigem Ammoniak auf 25 % werden davon 940 Teile für den nächsten Ansatz eingesetzt, ca. 35 Teile werden ausgeschleust.

Die Suspension (ca. 3100 Teile, $p_H$ 13,4) läuft in ca. 15 Minuten aus einem Tropftrichter in vorgelegte, außen gekühlte 1000 Teile ca. 30 %-ige Salzsäure (8,5 Mol). Dann wird zum Sieden erhitzt und das Schwefeldioxid in ca. 2 Stunden durch Kochen quantitativ ausgetrieben. Das Schwefeldioxid wird in einer Mischung von 156 Teilen Wasser und 255 Teilen 33 %iger Natronlauge aufgefangen. Man erhält 546 Teile einer 40 %-igen Natriumhydrogensulfitlösung (2,1 Mol entsprechend 93 % der Theorie), die wieder für den nächsten Ansatz verwendet werden.

Man erhält ca. 4000 Teile einer Suspension, die nach dem Diazotierwert 6,7 Gewichtsprozent = 268 Teile 2-Aminonaphthalin-6(8)-sulfonsäure enthält (80 % der Theorie bezogen auf 2-Hydroxynaphthalin) mit einem Gehalt von 5 ppm 2-Aminonaphthalin (HPTLC, Scanner).

Dann kühlt man die Suspension in ca. 2 Stunden auf 60°C ab und isoliert die Brönner-säure durch Filtration.

Darauf wäscht man mit ca. 2000 Teilen einer 2 %-igen Salzsäure bei 25°C bis zum farblosen Ablauf des Waschfiltrats. Man erhält 230 Teile reine 2-Aminonaphthalin-6-sulfonsäure 100 % gerechnet (Brönner-Säure) (= 1,03 Mol entsprechend 86 % der Theorie bezogen auf Diazotierwert der Suspension bzw. 69 % der Theorie, bezogen auf 2-Hydroxynaphthalin).

Der Reingehalt (Diazotierwert) beträgt 96,7 %. Das Produkt ist dünnachichtchromatographisch rein. Der Gehalt an 2-Aminonaphthalin ist < 100 ppm (HPTLC, Scanner). Man erhält außerdem 3500 Teile Mutterlauge ($d^{20}$ = 1,12, $p_H$ 0,5) mit

4,9 Teilen 2-Aminonaphthalin-6-sulfonsäure entsprechend 1,5 % der Theorie

11,2 Teilen 2-Aminonaphthalin-8-sulfonsäure entsprechend 3,3 % der Theorie

14 Teilen 2-Aminonaphthalindisulfonsäuren entsprechend 3,1 % der Theorie

(ermittelt mit HPTLC, Scanner).

Der Diazotierwert der Mutterlauge beträgt 0,8 % (Mol 223, entsprechend 10 % der Theorie). Der Gehalt an 2-Aminonaphthalin beträgt 1 ppm.

Ohne die Entfernung des 2-Hydroxynaphthalins aus der Lösung der 2-Hydroxynaphthalin-6-sulfonsäure durch Extraktion mit Toluol oder Xylol würde man eine 2-Aminonaphthalin-6-sulfonsäure erhalten, die ca. 1,9 Gewichtsprozent 2-Aminonaphthalin enthält.

## Beispiel 2

a) Verfährt man wie in Beispiel 1 beschrieben, extrahiert jedoch die Lösung der angefallenen wässrigen 2-Hydroxynaphthalinsulfonsäure mit Toluol statt mit Xylol, so erhält man ca. 4200 Teile einer Suspension, die nach dem Diazotierwert 6,4 %, entsprechend 267 Teile 2-Aminonaphthalin-6(8)-sulfonsäure enthält. Der Gehalt an 2-Aminonaphthalin beträgt 5 ppm (HPTLC, Scanner).

b) Die so erhaltene Suspension [1,20 Mol 2-Aminonaphthalin-6(8)-sulfonsäure, $p_H$ 0,8] wird mit 245 Teilen 33 %-iger Natronlauge bei 70°C auf $p_H$ 7,0 gestellt und mit 155 Teilen Essigsäureanhydrid (1,5 Mol) im Verlauf von 15 Minuten versetzt. Der $p_H$ sinkt hierbei auf 3,0 und die Brönner-Säure geht in Lösung. Die Acetylierung ist quantitativ. Man isoliert durch Aussalzen 289 Teile 2-Acetaminonaphthalin-6-sulfonsäure 100 % gerechnet (1,09 Mol entsprechend 73 % der Theorie, bezogen auf 2-Hydroxynaphthalin) mit einem Reingehalt von ca. 70 % (Diazotierwert nach Verseifung, Mol 265,3). Der Gehalt an 2-Acetaminonaphthalin beträgt 100 ppm (HPLC).

In den vereinigten Mutterlaugen und Waschfiltraten (ca. 4400 Teile) befinden sich ca. 0,3 Teile 2-Acetaminonaphthalin entsprechend ca. 70 ppm.

## Beispiel 3

Man verfährt, wie in Beispiel 1 a) beschrieben, führt jedoch die Extraktion mit einer Gegenstromextraktionskolonne mit 5 theoretischen Stufen durch. Volumenverhältnis Speiselösung (Lösung der 2-Hydroxynaphthalin-6(8)-sulfonsäure): m-Xylol = 2 : 1.

Der Gehalt an 2-Hydroxynaphthalin im Raffinat (2-Hydroxynaphthalin-6(8)-sulfonsäurelösung) beträgt 6 ppm, die 2-Hydroxynaphthalin-Extraktionsausbeute 99,5 %.

## Beispiel 4

Nan verfährt, wie in Beispiel 2 b) beschrieben, setzt jedoch eine nach Beispiel 1 b) erhaltene Suspension von Brönner-Säure ein. Man erhält eine 2-Acetaminonaphthalin-6-sulfonsäure mit dem Reingehalt von ca. 70 %. Der Gehalt an 2-Acetaminonaphthalin beträgt 23 ppm (HPLC).

In den vereinigten Mutterlaugen und Waschfiltraten (ca. 4700 Teile) befinden sich ca. 0,1 Teile 2-Acetaminonaphthalin entsprechend ca. 28 ppm.

## Beispiel 5

Eine gemäß Beispiel 1 a) hergestellte ca. 22 %-ige wäßrige Lösung von 2-Hydroxynaphthalinsulfonsäuren (1500 Teile) hat einen Gehalt von ca. 0,40 % 2-Hydroxynaphthalin (6,0 Teile 100 % gerechnet).

Die Lösung wird bei 60°C mit 90 Teilen Aktivkohlepulver 3 Stunden verrührt und heiß abgesaugt. Anschließend wäscht man mit 90 Teilen Wasser. Man erhält 1410 Teile Produktfiltrat mit einem Gehalt von 0,04 % 2-Hydroxynaphthalin (0,56 Teile 100 % gerechnet) und 307 Teilen 2-Hydroxynaphthalinsulfonsäure (1,37 Mol).

In der Kohle werden 5,4 Teile 2-Hydroxynaphthalin (90 % der ursprünglichen Menge) und 30 Teile 2-Hydroxynaphthalinsulfonsäure (0,13 Mol entsprechend ca. 9 % der Theorie) zurückgehalten. Mit 60 Teilen Aktivkohlepulver werden 83 % der ursprünglichen Menge 2-Hydroxynaphthalin (entsprechend 5 Teile) zurückgehalten.

## Beispiel 6

1410 Teile wäßrige 2-Hydroxynaphthalin-6(8)-sulfonsäure (enthaltend 307 Teile = 1,37 Mol), hergestellt gemäß Beispiel 5, werden in der in Beispiel 1 b) beschriebenen Weise in die 2-Aminonaphthalin-6-sulfonsäure überführt.

Man erhält ca. 4150 Teile einer Suspension, die nach dem Diazotierwert 6,0 % entsprechend 249 Teile 2 Aminonaphthalin-6(8)-sulfonsäure (1,11 Mol) enthält. Der Gehalt an 2-Aminonaphthalin ist 60 ppm.

Zum Unterschied von Beispiel 1 b) findet man hier auch 2-Aminonaphthalin im Ammoniak-Destillat (140 ppm).

## Beispiel 7

Die gemäß Beispiel 6 erhaltene Suspension, enthaltend 1,11 Mol 2-Aminonaphthalin-6(8)-sulfonsäure ($p_H$ - 1,0) wird, wie in Beispiel 2 b) beschrieben, in 2-Acetaminonaphthalin-6-sulfonsäure überführt. Man erhält nach dem Aussalzen 282 Teile 2-Acetaminonaphthalin-6-sulfonsäure 100 % gerechnet (1,06 Mol entsprechend 71 % der Theorie, bezogen auf 2-Hydroxynaphthalin, bzw. 77 % der Theorie bezogen auf 2-Hydroxynaphthalinsulfonsäure, bzw. 96 % der Theorie, bezogen auf 2-Aminonaphthalin-6(8)-sulfonsäure) mit einem Reingehalt von 88 % (aus Acetyl-Bestimmung). Der Gehalt an 2-Acetaminonaphthalin beträgt 965 ppm (HPLC).

In den vereinigten Mutterlaugen und Waschfiltraten (6250 Teile) befinden sich ca. 0,2 Teile 2-Acetaminonaphthalin entsprechend ca. 30 ppm.

## Beispiel 8

Eine gemäß Beispiel 1 a) hergestellte Sulfierschmelze wird in 1050 Teilen Wasser aufgenommen (1482 Teile) und nach Hochheizen zum Rückfluß auf 35°C abgekühlt. Sie enthält 0,21 % 2-Hydroxynaphthalin (3,1 Teile).

Man läßt 180 Teile 33 %-ige Natronlauge (1,50 Mol) hinzulaufen. Der $p_H$-Wert beträgt dann 0,4. Zu der dünnen, weißgrauen Suspension läßt man dann noch 74 Teile 25 %-iges wäßriges Ammoniak (1,08 Mol) hinzulaufen (der $p_H$-Wert beträgt anschließend 3,0). ·

Die bei 50°C dünne, weißgraue Suspension wird auf 95°C geheizt. Bei 88°C geht das Salzgemisch völlig in Lösung. Anschließend trägt man 60 Teile Aktivkohlepulver ein und rührt ca. 45 Minuten bei 95°C nach. Dann filtriert man über eine auf 95°C mit Streichdampf beheizte Drucknutsche von der Kohle unter Stickstoffdruck ab.

Das Produktfiltrat (1516 Teile) weist einen $p_H$-Wert von 7,5 auf. Es enthält 0,06 % 2-Hydroxynaphthalin, entsprechend 0,91 Teilen.

Es wurden nur 70 % des restlichen 2-Hydroxynaphthalins adsorbiert. Der Filterrückstand beträgt nach dem Trocknen bei 25°C 114 Teile. Seine Sulfatasche beträgt 7,0 %. Der Gehalt an 2-Hydroxynaphthalin beträgt 1,8 % entsprechend 2,1 Teilen, der Gehalt an 2-Hydroxynaphthalinsulfonsäure 25 Teile, entsprechend 7 % der Theorie (Titration von naphtholischem Hydroxyl).

Die Adsorption des 2-Hydroxynaphthalins aus der Salzlösung ist somit deutlich niedriger als aus der Sulfonsäurelösung (vergleiche hierzu Beispiel 5).

## Beispiel 9

1516 Teile gemäß Beispiel 8 erhaltene wäßrige 2-Hydroxynaphthalin-6(8)-sulfonsäure ($p_H$ 7,5) werden mit 500 Teilen Wasser verdünnt und auf 5°C abgekühlt. Man versetzt mit 600 Teilen ca. 40 %-iger Natriumhydrogensulfitlösung und 775 Teilen 25 %-igem Ammoniak ($p_H$ 10,6).

Die Suspension wird in einen VA-Rührautoklav überführt und, wie in Beispiel 1b beschrieben, in 2-Aminonaphthalin-6-sulfonsäure überführt.

Man erhält ca. 4380 Teile einer Suspension, die nach dem Diazotierwert 5,55 % entsprechend 243 Teile (1,09 Mol) 2-Aminonaphthalin-6(8)-sulfonsäure enthält, entsprechend 73 % der Theorie, bezogen auf 2-Hydroxynaphthalin. Der Gehalt an 2-Aminonaphthalin beträgt 83 ppm (vergleiche hierzu Beispiel 6).

Bezieht man diesen Wert auf die 243 Teile Brönner-Säure, so ergibt sich für dieses Produkt, falls es durch Filtration isoliert wird, ein Gehalt von 1500 ppm 1-Aminonaphthalin, der unerwünscht hoch ist.

## Beispiel 10

Die gemäß Beispiel 9 erhaltene Suspension [enthaltend 1,09 Mol 2-Aminonaphthalin-6(8)-sulfonsäure, $p_H$-Wert 0,9] wird, wie in Beispiel 2 b) beschrieben, in 2-Acetaminonaphthalin-6-sulfonsäure überführt.

Man erhält nach dem Aussalzen 247 Teile 2-Acetaminonaphthalin-6-sulfonsäure 100 % gerechnet (0,93 Mol entsprechend 86 % der Theorie, bezogen auf 2-Aminonaphthalin-6(8)-sulfonsäure (mit einem Reingehalt von 74 %, aus dem Diazotierwert nach Verseifung ermittelt). Der Gehalt an 2-Acetaminonaphthalin beträgt 2120 ppm (HPLC).

In den vereinigten Mutterlaugen und Waschfiltraten (5542 Teile) befinden sich 0,4 Teile 2-Acetaminonaphthalin entsprechend ca. 70 ppm.

## Beispiel 11

a) In einem Rührkolben werden 215 Teile Schwefelsäure 96 % (2,1 Mol) vorgelegt. Unter langsamem Rühren werden 216 Teile 2-Hydroxynaphthalin (1,5 Mol) in Form von Schuppen bei 30°C in ca. 30 Minuten eingetragen. Man heizt auf genau 86°C (der Kolben soll möglichst tief im Heizbad eingetaucht sein) und rührt 30 Minuten bei dieser Temperatur bis zum völligen Verschwinden des sich langsam lösenden Bodenkörpers aus 2-Naphthol. Dann heizt man auf 95°C in ca. 1,5 Stunden. Man evakuiert auf 1 - 1,5 mbar und entgast den Ansatz bei 95°C. Zur anschließenden Abdestillation von Wasser wird zunächst in 20 Minuten auf 100°C geheizt, dabei gehalten bis zum Nachlassen der Destillation und dann in 20 Minuten auf 106°C geheizt. Es bildet sich ein Hauch von Sublimat aus 2-Hydroxynaphthalin am obersten, kälteren Teil der Kolbeninnenwand.

Die Menge des abdestillierten Wassers in der Vorlage und Kältefalle beträgt 12 Teile.

Die dünnflüssige bis schwach viskose Schmelze (106°C) wird sofort anschließend in 840 Teile Wasser eingerührt, wobei sich die Temperatur auf 45°C einstellt. Der im Sulfierkolben verbliebene Anteil wird mit 150 Teilen Wasser auf einmal aufgenommen und nach kräftigem Durchrühren ebenfalls zugemischt. Man spült den Sulfierkolben mit 60 Teilen Wasser und gibt diese zum Ansatz. Dann heizt man die trübe, schwarze Lösung zum Rückfluß und hält maximal 15 Minuten am Rückfluß.

Die ca. 22 %-ige Produktlösung (d20 = 1,115, Menge ca. 1475 Teile) hat einen Gehalt von 0,13 % 2-Hydroxynaphthalin, entsprechend 1,92 Teilen 100 %, entsprechend 0,9 % d.Th. (HPTLC). Durch die Abdestillation von Wasser werden also zusätzlich zum Ergebnis des Beispiel 1 noch 2,6 Teile (1,2 % d.Th.) 2-Naphthol umgesetzt.

Die Produktlösung wird mit 260 Teilen m-Xylol bei 80°C einmal ausgerührt. Die extrahierte Produktlösung (1464 Teile) enthält 323 Teile 2-Hydroxynaphthalin-6(8)-sulfonsäure (1,44 Mol) und hat einen Gehalt von 0,07 % 2-Hydroxynaphthalin, entsprechend 1,03 Teilen 100 %, entsprechend 0,5 % d.Th. (HPTLC). Sie wird bei 50°C nacheinander 2 mal mit je 30 Teilen A-Kohlepulver verrührt und geklärt. Der Filterrückstand wird jeweils mit 300 Teilen Wasser gewaschen. Der Gehalt an 2-Naphthol sinkt dabei auf 62 ppm (0,105 Teile, 0,05 % d.Th.) im 1. Filtrat (1700 Teile) bzw. auf 5 ppm (0,01 Teile) im 2. Filtrat (2000 Teile).

Bei der umgekehrten Arbeitsweise (erst Klären über Kohle, dann 1 mal Ausrühren mit m-Xylol) beträgt der Gehalt an 2-Naphthol noch 100 ppm (entsprechend 0,17 Teile).

b) 2000 Teile der, wie vorstehend (Absatz a) beschrieben, hergestellten wäßrigen Lösung der 2-Hydroxynaphthalin-6(8)-sulfonsäure (325 Teile = 1,45 Mol) werden mit 196 Teilen 25 %-igem wäßrigem Ammoniak (2,88 Mol) unter Außenkühlung auf pH 7,8 gestellt. Die Suspension wird bei 5°C nacheinander mit 620 Teilen ca. 40 %-iger Natriumhydrogensulfitlösung (2,35 Mol), 188 Teilen 33 %-iger Natronlauge (1,55 Mol) und schließlich 775 Teilen 25 %-igem wäßrigem Ammoniak (11,38 Mol) versetzt.

Die Suspension (pH 12,6) wird in einem VA-Rührautoklav in ca. 40 Minuten auf 160°C erhitzt und 8 Stunden bei dieser Temperatur nachgerührt. Der Druck beträgt konstant ca. 9 - 10 bar.

Dann werden über eine Druckschleuse bei 20°C unter Stickstoffdruck 651 Teile 33 %-ige Natronlauge (5,37 Mol) aufgedrückt. Der Ansatz wird 1 Stunde bei 100°C nachgerührt. Nan kühlt auf 20°C ab, entspannt vorsichtig und entleert den Autoklav. Anschließend spült man mit 200 Teilen Wasser nach. Erhalten werden 4460 Teile einer dünnen, hellbraunen Suspension der 2-Aminonaphthalin-6(8)-sulfonsäure (Salz) (pH 13,4). Darauf destilliert man 868 Teile ca. 18 %-iges wäßriges Ammoniak (ca. 75 % der Theorie) bei Normaldruck bis zu einer Kopftemperatur von 100°C ab (Badtemperatur bis 150°C, Kältefalle als Vorlage). Das Ammoniak-Destillat ist durch Xylol-Tröpfchen schwach trüb und enthält gemäß HPTLC kein 2-Aminonaphthalin (< 3 ppm).

Nach Aufstärken mit 77 Teilen gasförmigem Ammoniak auf 25 % werden davon 775 Teile für den nächsten Ansatz eingesetzt, ca. 170 Teile werden ausgeschleust.

Die Suspension (3600 Teile, pH 13,4) läuft in ca.

15 Minuten aus einem Tropftrichter in vorgelegte, außen gekühlte 1034 Teile ca. 30 %-iger Salzsäure (8,8 Mol) (→ pH 0,6). Dann wird zum Sieden erhitzt und das Schwefeldioxid in ca. 2 Stunden durch Kochen quantitativ ausgetrieben. Das Schwefeldioxid wird in einer Mischung von 164 Teilen Wasser und 267 Teilen 33 %-iger Natronlauge aufgefangen. Man erhält 572 Teile einer 40 %-igen Natriumhydrogensulfitlösung (2,2 Mol entsprechend 93 % der Theorie), die wieder für den nächsten Ansatz verwendet werden. Man erhält 4730 Teile einer Suspension, die nach dem Diazotierwert 6,1 Gewichtsprozent (287,5 Teile) 2-Aminonaphthalin-6(8)-sulfonsäure enthält (1,287 Mol = 85,8 % der Theorie, bezogen auf 2-Hydroxynaphthalin) mit einem Gehalt von 3 ppm 2-Aminonaphthalin (HPTLC, Scanner).

c) Die so erhaltene Suspension (gemäß Diazotierwert 4730 Teile 6,1 %-ig = 1,287 Mol 2-Aminonaphthalin-6(8)-sulfonsäure), wird mit 314 Teilen 33 %-iger Natronlauge bei 70°C auf pH 7,0 gestellt und mit 0,3 Teilen eines Entschäumers (fluorierte organische Verbindung) versetzt. Dann laufen unter Rühren 158 Teile Essigsäureanhydrid (1,54 Mol) im Verlauf von 15 Minuten hinzu.

Der pH sinkt hierbei auf 3,2 und die Brönner-Säure geht in Lösung. Die Acetylierung ist quantitativ (DC u. Nitrosierung). Man isoliert durch Aussalzen 277 Teile isomerenfreie 2-Acetaminonaphthalin-6-sulfonsäure 100 % gerechnet (= 1,04 Mol entsprechend 81 % d.Th. bezogen auf den Diazotierwert bzw. 69,3 % d.Th., bezogen auf 2-Hydroxynaphthalin) mit einem Reingehalt von ca. 70 % (Acetylbestimmung u. Kohlenstoffanalyse, Mol 265,3, HPTLC).

Der Gehalt an 2-Acetaminonaphthalin beträgt ca. 50 ppm (HPLC).

Die Mutterlauge enthält neben dem Zielprodukt auch noch die isomere 2-Acetaminonaphthalin-8-sulfonsäure (ca. 4 Mol %) (HPTLC) und Spuren 2-Acetaminonaphthalin (< 0,3 Teile).

## Patentanspruch

Eintopfverfahren zur Herstellung von 2-Acetaminonaphtalin-6-sulfonsäure hoher Reinheit durch Sulfonierung von 2-Hydroxynaphthalin mit konzentrierter Schwefelsäure, Überführung der gebildeten 2-Hydroxynaphthalinsulfonsäure mit Ammoniak in Gegenwart von Ammoniumhydrogensulfit in die 2-Aminonaphthalin-6-sulfonsäure (Bucherer-Reaktion) und deren N-Acetylierung zur 2-Acetaminonaphthalin-6-sulfonsäure, dadurch gekennzeichnet, daß man nach Verdünnung der Sulfierschmelze mit Wasser in der angefallenen wäßrigen Lösung der 2-Hydroxynaphthalin-6-sulfonsäure noch enthaltene Verunreinigungen, insbesondere 2-Hydroxynaphthalin, durch Extraktion mit Toluol oder Xylol und/oder Klären über Aktivkohle weitestgehend entfernt.

**Claim**

A single-vessel process for preparing 2-acetaminonaphthalene-6-sulfonic acid of high purity by sulfonating 2-hydroxynaphthalene with concentrated sulfuric acid, converting the 2-hydroxynaphthalenesulfonic acid formed with ammonia in the presence of ammonium hydrogensulfite into 2-aminonaphthalene-6-sulfonic acid (Bucherer reaction) and N-acetylating the latter to give 2-acetaminonaphthalene-6-sulfonic acid, which comprises, after diluting the sulfonating melt with water, substantially removing any impurities still present in the resulting aqueous solution of 2-hydroxynaphthalene-6-sulfonic acid, in particular 2-hydroxynaphthalene, by extraction with toluene or xylene and/or clarification using active carbon.

**Revendication**

Procédé en un pot pour la préparation d'acide acétamino-2 naphtalènesulfonique-6 de haute pureté, par sulfonation d'hydroxy-2 naphtalène avec de l'acide sulfurique concentré, conversion de l'acide hydroxy-2 naphtalènesulfonique ainsi formé, avec de l'ammoniac en présence d'hydrogénosulfite d'ammonium, en l'acide amino-2 naphtalènesulfonique-6 (réaction de Bucherer), et sa N-acétylation en acide acétamino-2 naphtalènesulfonique-6, caractérisé en ce que, après dilution, à l'eau, du bain fondu de sulfonation, on élimine en presque totalité, par extraction au toluène ou au xylène et/ou clarification sur charbon actif, les impuretés contenues encore dans la solution aqueuse obtenue de l'acide hydroxy-2 naphtalènesulfonique-6, et en particulier l'hydroxy-2 naphtalène.